# EUROPÄISCHE PATENTSCHRIFT

(11) **EP 3 359 646 B1**
(45) Veröffentlichungstag und Bekanntmachung des Hinweises auf die Patenterteilung: **17.03.2021**
(21) Anmeldenummer: 16774451.5
(22) Anmeldetag: 22.09.2016
(51) Int. Cl.: C12N 5/075, C12N 5/0735

(54) **POLKÖRPERINJEKTION**
POLAR BODY INJECTION
INJECTION DE GLOBULES POLAIRES

(30) Priorität: 09.10.2015 DE 102015013156
(43) Veröffentlichungstag der Anmeldung: 15.08.2018
(73) Patentinhaber: Würfel, Wolfgang, 85235 Sixtnitgern (DE); Würfel, Franziska, 85235 Sixtnitgern (DE)
(72) Erfinder: WÜRFEL, Wolfgang, 85235 Sixtnitgern (DE)
(74) Vertreter: Samson & Partner Patentanwälte mbB
(86) Internationale Anmeldenummer: PCT/EP2016/001588
(87) Internationale Veröffentlichungsnummer: WO 2017/059946

(56) Entgegenhaltungen:
- WO-A1-2008/033469
- WO-A2-03/100018
- WO-A2-2004/003182
- DE-A1-102004 062 184
- US-A1- 2003 129 745
- US-A1- 2014 335 619
- SAYAKA WAKAYAMA ET AL: "Efficient establishment of mouse embryonic stem cell lines from single blastomeres and polar bodies", STEM CELLS, ALPHAMED PRESS, DAYTON, OH, US, Bd. 25, Nr. 4, 1. April 2007 (2007-04-01), Seiten 986-993, XP002635116, ISSN: 1066-5099, DOI: 10.1634/STEMCELLS.2006-0615 [gefunden am 2006-12-21]
- JU J Y ET AL: "Establishment of stem cell lines from nuclear transferred and parthenogenetically activated mouse oocytes for therapeutic cloning", FERTILITY AND STERILITY, ELSEVIER SCIENCE INC, NEW YORK, NY, USA, Bd. 89, Nr. 5, 1. Mai 2008 (2008-05-01), Seiten 1314-1323, XP022668260, ISSN: 0015-0282, DOI: 10.1016/J.FERTNSTERT.2006.11.203 [gefunden am 2007-11-05]

## Beschreibung

### Technisches Gebiet

Die vorliegende Erfindung betrifft im Allgemeinen das Gebiet der Stammzellgewinnung und insbesondere ein Verfahren zur Injektion eines Polkörpers in eine Eizelle.

### Hintergrund

Stammzellen haben die Fähigkeit, durch Differenzierung ein breites Spektrum an verschiedenen Zelltypen hervorzubringen. Aufgrund dieser Fähigkeit zur Differenzierung besteht an der Gewinnung und Verwendung von Stammzellen ein Interesse aus medizinischer Sicht, zum Beispiel um krankes oder verletztes Gewebe zu ersetzen.

Verschiedene Verfahren zur Gewinnung von Stammzellen haben ethische Bedenken genährt, insbesondere bezüglich der Verwendung von Embryonen.

### Aufgabenstellung

Vor diesem Hintergrund ist es eine Aufgabe der Erfindung Verfahren zur Stammzellgewinnung ohne Verwendung von Embryonen oder embryonaler Stammzellen bereitzustellen.

### Kurzbeschreibung

Diese Aufgabe wird gelöst durch ein Verfahren und Stammzellen gemäß der unabhängigen Patentansprüche. Die abhängigen Ansprüche beschreiben bevorzugte Ausführungsformen.

Ein erfindungsgemäßes Verfahren umfasst das Entnehmen eines Polkörpers aus einer ersten Eizelle und das Injizieren des Polkörpers in eine sich in einem befruchtungsfähigen Zustand befindende zweite Eizelle.

Unter dem Begriff "Polkörper" soll hier insbesondere der so genannte sekundäre Polkörper erfasst sein, welcher mit haploidem Chromosomensatz während der zweiten Reifeteilung, Meiose II, gebildet wird. Falls ein solcher Polkörper nicht erfindungsgemäß entnommen wird, sondern vorerst in der Eizelle verbleibt, so kann er im Allgemeinen im Rahmen der Meiose II ausgestoßen werden.

Hier soll unter dem Begriff "Eizelle" neben dem reifen, befruchtungsfähigen Ovum ferner mindestens der sich im Rahmen der Oogenese in der Reifung befindliche Oozyt erfasst sein.

Unter dem Begriff "befruchtungsfähiger Zustand" soll insbesondere verstanden werden, dass die Eizelle (a) bis zum Zustand des Ovums gereift ist und (b) noch nicht befruchtet wurde; weiterhin dass diese Eizelle ihre Polkörper bereits ausgestoßen hat. Demgegenüber führt die Befruchtung einer Eizelle dazu, dass sie im Allgemeinen geeignet sein kann, den Prozess der Entwicklung eines Organismus, z.B. eines Menschen, in Gang zu setzen. Das erfindungsgemäße Verfahren verwendet weder Rohmaterial noch Zwischenprodukte, welche geeignet wären, den Prozess der Entwicklung eines Organismus in Gang zu setzen. Insbesondere verwendet das Verfahren keine befruchtete Eizellen. Auch werden weder männliche Keimzellen noch Chromosomen oder Erbgut männlichen Ursprungs verwendet. Durch das Injizieren des Polkörpers in die zweite Eizelle kann stattdessen beispielsweise eine unimaternale Disomie aller Chromosomen entstehen, welche nach derzeitigen Erkenntnissen nicht imstande ist, sich zu einem lebensfähigen Organismus zu entwickeln.

Insbesondere ist es vorgesehen, dass das Entnehmen des Polkörpers und das Injizieren des Polkörpers in vitro durchgeführt werden. Bei solchen Ausführungsformen kann die erste Eizelle einem ersten Individuum entnommen worden sein. Die zweite Eizelle kann dem ersten Individuum oder einem zweiten Individuum entnommen worden sein. Das Entnehmen der ersten Eizelle aus einem ersten Individuum und das Entnehmen der zweiten Eizelle aus dem ersten Individuum oder aus einem zweiten Individuum können von Ausführungsformen des erfindungsgemäßen Verfahrens umfasst sein.

Im Allgemeinen können die Eizellen aus jeglichem Organismus entnommen worden sein. In einigen Ausführungsformen können die erste Eizelle und die zweite Eizelle jeweils humane Eizellen sein.

Es handelt sich bei der ersten Eizelle und der zweiten Eizelle um zwei separate Eizellen.

Im Allgemeinen kann das Entnehmen eines Polkörpers aus der ersten Eizelle mindestens Fixieren der ersten Eizelle, Öffnen der ersten Eizelle und/oder Absaugen des Polkörpers aus der ersten Eizelle umfassen.

Im Allgemeinen kann das Injizieren des Polkörpers intrazytoplasmatisch erfolgen. Unter dem Begriff "intrazytoplasmatische Injektion" soll eine Injektion in das Innere des Zytoplasmas bezeichnet sein. Durch intrazytoplasmatische Injektion wird der Polkörper in Nähe der Chromosomen der zweiten Eizelle gebracht.

Alternativ oder zusätzlich kann das Injizieren des Polkörpers (i) das Ablegen des Polkörpers zwischen der zona pellucida der zweiten Eizelle und dem Zytoplasma der zweiten Eizelle sowie (ii) die Verschmelzung der Membranen, beispielsweise mittels Elektrofusion, umfassen. Vorzugsweise wurde zumindest vor Verschmelzung der Membranen ein von der zweiten Eizelle ausgestoßener Polkörper entfernt. In Folge der Elektrofusion kann der Inhalt des injizierten Polkörpers, insbesondere die enthaltenen Chromosomen, in das Zytoplasma und somit in Nähe der Chromosomen der zweiten Eizelle gelangen.

Im Allgemeinen kann das Verfahren zusätzlich ein Lysieren, d.h. das Herbeiführen einer Lyse, des Polkörpers umfassen. Das Lysieren des Polkörpers kann insbesondere zwischen dessen Entnahme und Injektion erfolgen. In diesem Fall wird anstatt des intakten Polkörpers der lysierte Polkörper injiziert. Mit "lysierter Polkörper" soll im Folgenden zumindest das Lysat des Polkörpers bezeichnet sein. Der lysierte Polkörper umfasst insbesondere die Chromosomen des entnommenen Polkörpers. Für das Injizieren des lysierten Polkörpers kann im Allgemeinen eine Injektionspipette mit geringem Durchmesser verwendet werden, selbst wenn das Injizieren des entnommenen Polkörpers im intakten Zustand in manchen Ausführungsbeispielen mit der Injektionspipette mit geringem Durchmesser nicht möglich wäre. In bevorzugten Ausführungsformen kann das Lysieren des Polkörpers osmotisch erfolgen. Hierzu kann der Polkörper in einer hypoosmolaren Lösung platziert werden. Die hypoosmolare Lösung kann sich beispielsweise unter Ölverschluss befinden. Durch Osmose kann der Polkörper somit zum "Platzen", d.h. zur Lyse, gebracht werden. Durch Aufnahme der hypoosmolaren Lösung in die Injektionspipette kann auch der lysierte Polkörper in die Injektionspipette aufgenommen werden.

Im Allgemeinen kann das Verfahren zusätzlich das Kultivieren der zweiten Eizelle bis zur Bildung einer Blastozyste und das Isolieren mindestens einer Stammzelle aus der Blastozyste umfassen. Die mindestens eine Stammzelle kann vorzugsweise aus einer inneren Zellmasse der Blastozyste isoliert werden. Beispielsweise ist das Isolieren der mindestens einen Stammzelle aus einer Blastozyste in der Patentschrift DE 10 2004 062 184 B4 gezeigt. Im Allgemeinen kann es sich bei der mindestens einen isolierten Stammzelle um eine embryonale Stammzelle handeln.

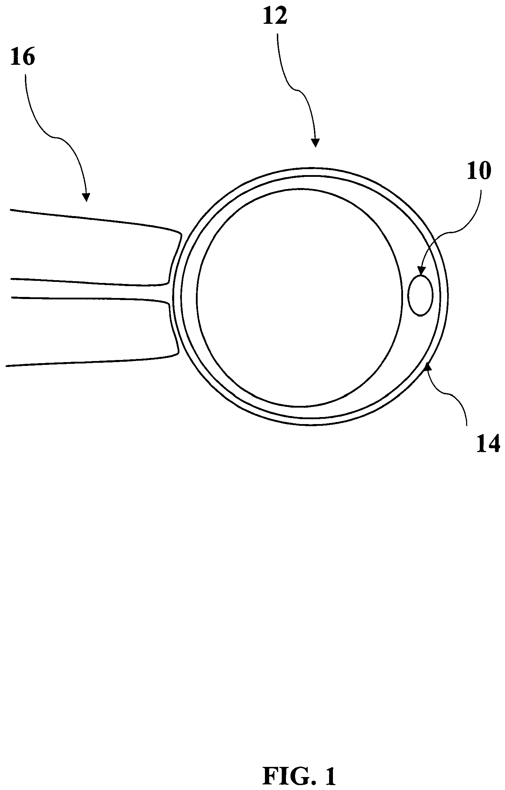

SAYAKA WAKAYAMA ET AL: "Efficient establishment of mouse embryonic stem cell lines from single blastomeres and polar bodies", STEM CELLS, ALPHAMED PRESS, DAYTON, OH, US, Bd. 25, Nr. 4, 1. April 2007 (2007-04-01), Seiten 986-993 beschreibt die Gewinnung von pluripotenten Stammzellen von Blastomeren und Polkörpern (Abb. 1). Polkörper werden von einer ersten Eizelle entnommen und in eine entkernte Eizellle injiziert. Die rekonstruierte Eizelle wird aktiviert sodass der 2. Polkörper in der Zelle bleibt. Die erhaltenen Zellen werden bis zum Blastozysten Stadium kultiviert, sodass aus ihnen Stammzellen gewonnen werden können. WO 2004/003182, US 2003/129745, und WO 2008/033469 beschreiben weitere Verfahren zur Gewinnung von Stammzellen aus Oozyten.

### Kurzbeschreibung der Zeichnungen

In der folgenden Beschreibung von Ausführungsbeispielen der Erfindung wird auf die beigefügten Zeichnungen Bezug genommen, die zeigen:
FIG. 1 eine schematische Darstellung einer ersten Eizelle vor Entnehmen des Polkörpers,
FIG. 2 eine schematische Darstellung einer ersten Eizelle während Entnehmen des Polkörpers,
FIG. 3 eine schematische Darstellung einer zweiten Eizelle kurz vor Injizieren eines Polkörpers,
FIG. 4 eine schematische Darstellung einer zweiten Eizelle nach Injizieren eines Polkörpers,
FIG. 5 eine schematische Darstellung einer zur Blastozyste gereiften zweiten Eizelle während Entnehmen einer Stammzelle.
FIG. 6 eine schematische Darstellung einer weiteren Ausführungsform einer zweiten Eizelle während der Injektion eines Polkörpers.

### Detaillierte Beschreibung

FIG. 1 zeigt eine schematische Darstellung einer ersten Eizelle 12 vor Entnehmen des Polkörpers 10. Die erste Eizelle 12 ist mit Hilfe einer Haltepipette 16 fixiert. Die Haltepipette 16 ist im vorliegenden Fall von der in der Darstellung linken Seite an die erste Eizelle 12 herangeführt worden und die erste Eizelle wurde soweit gedreht, dass sich der Polkörper 10 auf der in der Darstellung rechten Seite der ersten Eizelle 12 befindet. Die erste Eizelle umfasst unter anderem eine Plasmamembran 14, welche auch den Polkörper 10 umschließt.

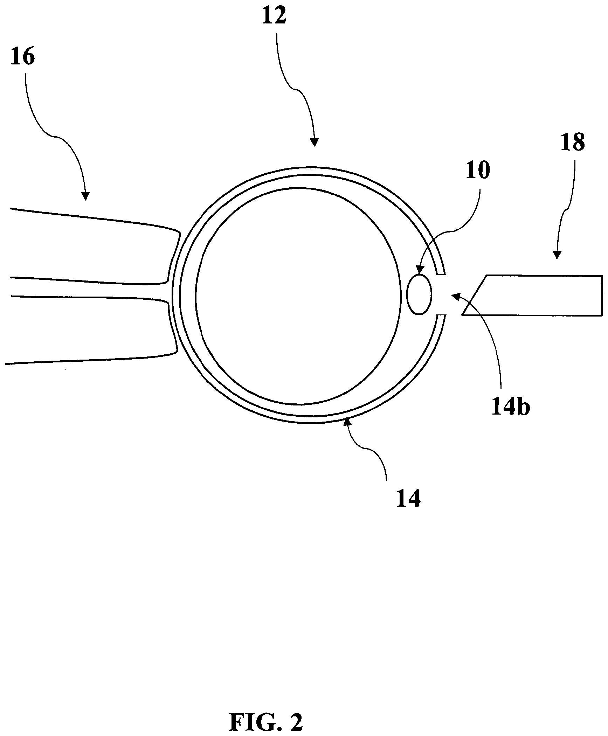

FIG. 2 zeigt eine schematische Darstellung einer ersten Eizelle 12 während Entnehmen des Polkörpers 10. Es kann sich insbesondere um die in FIG. 1 dargestellte erste Eizelle 12 handeln. Die Plasmamembran 14 der ersten Eizelle 12 weist eine Öffnung 14b auf, welche beispielsweise mit Hilfe eines Lasers hergestellt wurde. Die Öffnung 14b befindet sich in Nähe des zu entnehmenden Polkörpers 10. Durch die Öffnung 14b kann eine Biopsie-Pipette 18 eingeführt werden. Die dargestellte Biopsie-Pipette 18 befindet sich unmittelbar vor Einführung in die Öffnung 14b. Die Biopsie-Pipette 18 dient dazu, den Polkörper abzusaugen und somit aus der ersten Eizelle 12 zu entnehmen. Im Anschluss an das Entnehmen kann der Polkörper bis zur weiteren Verwendung im Kulturmedium gelagert werden.

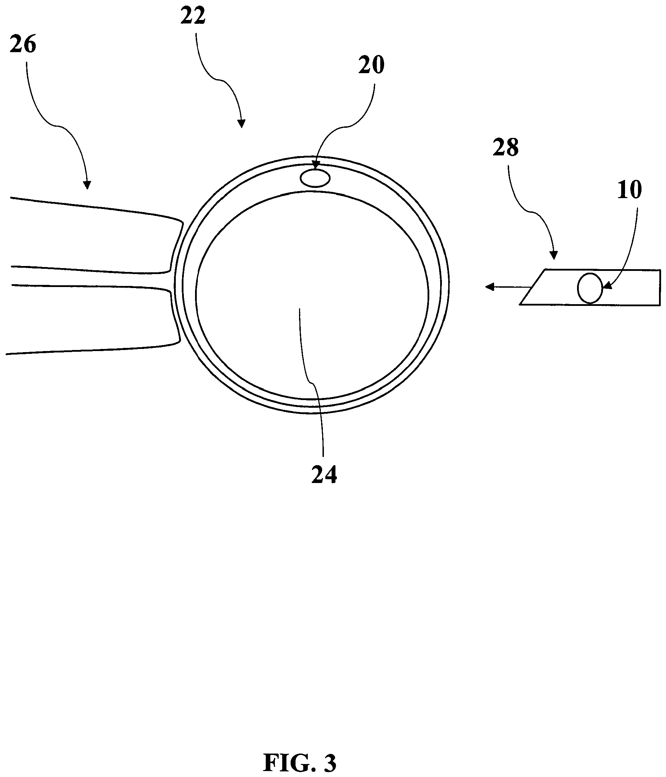

FIG. 3 zeigt eine schematische Darstellung einer zweiten Eizelle 22 kurz vor Injizieren eines Polkörpers 10. Der Polkörper 10 kann beispielsweise aus einer ersten Eizelle 12, wie in FIG. 1 und/oder FIG. 2 dargestellt entnommen worden sein. Die zweite Eizelle 22 umfasst unter anderem ein Zytoplasma 24. Der durch die zweite Eizelle 22 im Zuge ihrer Reifung ausgestoßene Polkörper ist mit Bezugszeichen 20 gekennzeichnet. Dieser befindet sich im vorliegenden Fall vorzugsweise in der Darstellung am oberen Rand ("auf 12 Uhr") oder am unteren Rand ("auf 6 Uhr") um ein Schädigen der Spindel der zweiten Eizelle durch eine Injektionspipette zu vermeiden. Die Anwesenheit dieses ausgestoßenen Polkörpers ist jedoch nicht zwingend. So kann in manchen Ausführungsbeispielen der Polkörper in die selbe Eizelle injiziert werden, aus der er entnommen wurde. In diesen Fällen weist die selbe Eizelle nach Entnahme ihres Polkörpers keinen weiteren Polkörper auf.

Die zweite Eizelle ist mit Hilfe einer Haltepipette 26 fixiert. Im vorliegenden Fall ist die Eizelle auf der in der Darstellung linken Seite fixiert. Das Injizieren des Polkörpers 10 kann beispielsweise von der in der Darstellung rechten Seite erfolgen. Der Polkörper 10 befindet sich bereits in einer Injektionspipette 28. Er kann beispielweise aus einem Kulturmedium aufgenommen worden sein, in das er nach Entnehmen aus einer ersten Eizelle gelagert wurde. Die Injektionspipette 28 dient dazu, die Plasmamembran der zweiten Eizelle 22 zu rupturieren und bis ins Zytoplasma 24 zu ragen. Im Zytoplasma 24 kann dann der Polkörper 10 mit Hilfe der Injektionspipette 28 abgelegt werden.

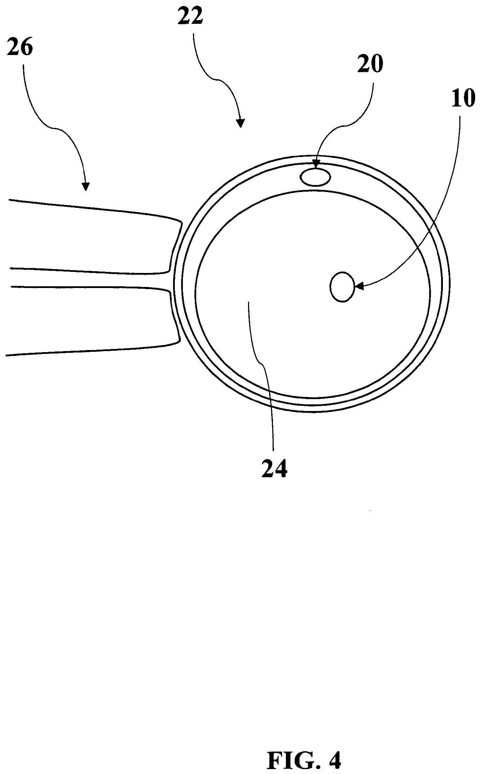

FIG. 4 zeigt eine schematische Darstellung einer zweiten Eizelle 22 nach intrazytoplasmatischem Injizieren eines Polkörpers 10. Die zweite Eizelle ist mit Hilfe einer Haltepipette 26 fixiert. Der Polkörper 10 befindet sich im Inneren des Zytoplasmas 24. Die zweite Eizelle 22 und der Polkörper 10 weisen je einen haploiden Chromosomensatz auf (nicht dargestellt). Durch Verschmelzen dieser beiden Chromosomensätze entsteht somit ein diploider Chromosomensatz. Beide Chromosomensätze sind mütterlichen Ursprungs, ein väterlicher Chromosomensatz ist nicht vorhanden. Es findet daher kein Verschmelzen von väterlichen und mütterlichen Chromosomen statt. Falls sowohl die erste Eizelle als auch die zweite Eizelle aus einem ersten Individuum entnommen sind, so handelt es sich bei der diploiden Zelle um eine unimaternale Disomie.

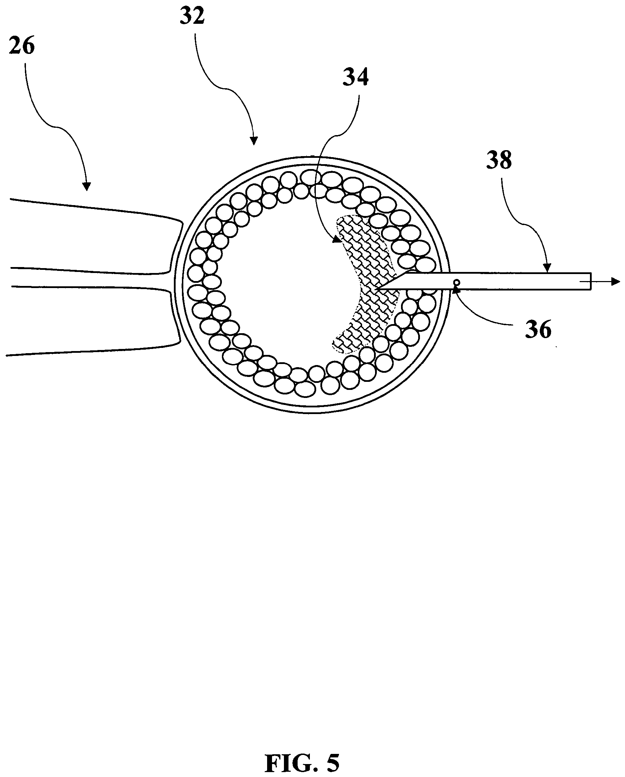

FIG. 5 zeigt eine schematische Darstellung einer Blastozyste 32. Die dargestellte Blastozyste 32 ist aus einer zweiten Eizelle herangereift, nachdem in die zweite Eizelle ein aus einer ersten Eizelle entnommener Polkörper injiziert wurde. Die Blastozyste 32 ist mit Hilfe einer Haltepipette 26 fixiert. Die Blastozyste 32 umfasst unter anderem eine innere Zellmasse 34. Mit Hilfe einer Pipette 38 kann mindestens eine Stammzelle 36 aus der inneren Zellmasse 34 entnommen und isoliert werden. Die mindestens eine isolierte Stammzelle kann im Folgenden zur Differenzierung angeregt werden um beispielsweise Gewebe für medizinische Zwecke zu generieren.

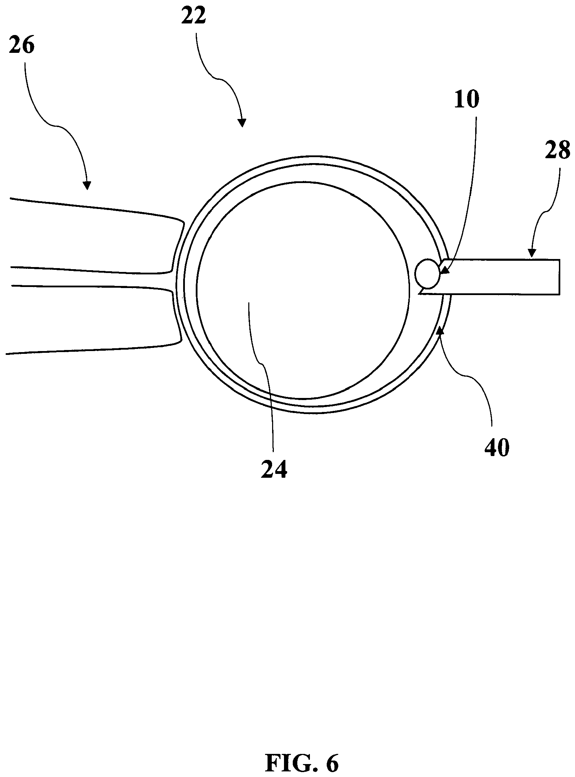

FIG. 6 zeigt eine schematische Darstellung einer weiteren Ausführungsform einer zweiten Eizelle 22 während der Injektion eines Polkörpers 10. Die zweite Eizelle 22 umfasst ein Zytoplasma 24 und eine zona pellucida 40. In dem dargestellten Ausführungsbeispiel wird der Polkörper mittels einer Injektionspipette 28 zwischen der zona pellucida 40 der zweiten Eizelle 22 und dem Zytoplasma 24 der zweiten Eizelle 22 abgelegt.

Daraufhin können die das Zytoplasma 24 umfangende Membran und die den Polkörper 10 umfangende Membran zur Verschmelzung angeregt werden. Dies geschieht bevorzugt mittels Elektrofusion. In Folge der Elektrofusion gelangt der Inhalt des injizierten Polkörpers 10, insbesondere die enthaltenen Chromosomen (nicht dargestellt), ins Zytoplasma 24 der zweiten Eizelle 22. In dem vorliegenden Ausführungsbeispiel wurde - im Gegensatz zu dem in Fig. 3 dargestellten Ausführungsbeispiel - zuvor ein von der zweiten Eizelle 22 ausgestoßener Polkörper entfernt und der Polkörper 10 an dessen Position platziert.

### Beispiele

Eine erste humane Eizelle im Oozyten-Stadium wird mit Hilfe einer Haltepipette (Saugkanüle mit Außendurchmesser ca. 100 µm, Innendurchmesser ca. 20 µm) unter einem Stereomikroskop fixiert. Das Fixieren geschieht derart, dass sich der Polkörper der ersten humanen Eizelle rechterhand, bei 3 Uhr in der Draufsicht, befindet. Mit Hilfe eines Laser (z.B. Infrarot-Laser, insbesondere bei 1,48 µm) wird an dieser Stelle eine Öffnung in der zona pellucida erzeugt. Der Polkörper wird mit Hilfe einer Biopsie-Pipette (Außendurchmesser ca. 15 µm, Innendurchmesser ca. 13 µm) abgesaugt. Der Polkörper wird in der gleichen Kulturschale in einen Kulturtropfen gelegt.

Eine zweite humane Eizelle, welche sich im reifen Ovum-Stadium befindet, wird mit Hilfe einer Haltepipette unter der Stereomikroskop fixiert. Mit Hilfe einer Injektionspipette wird der Polkörper aspiriert, die Plasmamembran rupturiert und der Polkörper tief in dem Zytoplasma der zweiten humanen Eizelle abgelegt. Die Injektionspipette wird zurückgezogen. Die Kultivierung der zweiten humanen Eizelle erfolgt gemäß Protokollen, wie der Fachmann sie aus dem Gebiet der in-vitro-Fertilisation oder der intrazytoplasmatischen Spermieninjektion (ICSI) kennt.

Nach Kultivierung der zweiten humanen Eizelle bis hin zum Blastozystenstadium erfolgt das Isolieren einer Stammzelle aus der Blastozyste gemäß dem in der Patentschrift DE 10 2004 062 184 B4 offenbarten Verfahren.

## Patentansprüche

1. Verfahren zur Gewinnung von Stammzellen,
**dadurch gekennzeichnet, dass**
das Verfahren umfasst:
- Entnehmen eines Polkörpers aus einer ersten Eizelle,
- Injizieren des Polkörpers in eine sich in einem befruchtungsfähigen Zustand befindende zweite Eizelle, welche nicht die erste Eizelle ist, und
- Kultivieren der zweiten Eizelle bis zur Bildung einer Blastozyste.

2. Verfahren nach einem der vorstehenden Ansprüche, bei welchem die erste Eizelle und die zweite Eizelle jeweils humane Eizellen sind.

3. Verfahren nach einem der vorstehenden Ansprüche, zusätzlich umfassend:
- Isolieren mindestens einer Stammzelle aus der Blastozyste.

4. Verfahren nach einem der vorstehenden Ansprüche, bei welchem das Entnehmen eines Polkörpers mindestens eines der folgenden Schritte umfasst: Fixieren der ersten Eizelle, Öffnen der ersten Eizelle, Absaugen des Polkörpers aus der ersten Eizelle.

5. Verfahren nach einem der vorstehenden Ansprüche, bei welchem das Injizieren des Polkörpers intrazytoplasmatisch erfolgt.

6. Verfahren nach einem der vorstehenden Ansprüche, wobei das Verfahren zusätzlich ein Lysieren, insbesondere ein osmotisches Lysieren, des Polkörpers umfasst.

## Claims

1. A method for stem cell production,
**characterized in that**
the method comprises:
- removing a polar body from a first egg cell,
- injecting the polar body into a second egg cell, which is in a fertile state and which is not the first egg cell, and
- cultivating the second egg cell up to the formation of a blastocyst.

2. A method according to one of the preceding claims, in which the first egg cell and the second egg cell each involve human egg cells.

3. A method according to one of the preceding claims, further comprising:
isolating at least one stem cell from the blastocyst.

4. A method according to one of the preceding claims, in which the removal of a polar body comprises at least one of the following steps: fixing the first egg cell, opening the first egg cell, aspirating the polar body from the first egg cell.

5. A method according to one of the preceding claims, in which the injection of the polar body is performed in intracytoplasmic manner.

6. A method according to one of the preceding claims, the method also comprising lysing, in particular osmotic lysing, of the polar body.

## Revendications

1. Procédé destiné à prélever des cellules souches,
**caractérisé en ce que**
le procédé comprend :
- l'extraction d'un corps polaire hors d'un ovocyte,
- l'injection du corps polaire dans un second ovocyte se trouvant dans un état disposé à la fécondation, lequel ovocyte n'est pas le premier ovocyte, et
- culture du second ovocyte jusqu'à former un blastocyste.

2. Procédé selon l'une des revendications précédentes, dans lequel le premier ovocyte et le second ovocyte sont respectivement des ovocytes humains.

3. Procédé selon l'une des revendications précédentes, comprenant en outre :
- l'isolement d'au moins une cellule souche hors du blastocyste.

4. Procédé selon l'une des revendications précédentes, dans lequel l'extraction d'un corps polaire comprend au moins l'une des étapes suivantes : fixation du premier ovocyte, ouverture du premier ovocyte, aspiration du corps polaire hors du premier ovocyte.

5. Procédé selon l'une des revendications précédentes, dans lequel l'injection du corps polaire se produit de façon intracytoplasmique.

6. Procédé selon l'une des revendications précédentes, dans lequel le procédé comprend en outre une lyse du corps polaire, en particulier une lyse osmotique.
